# EUROPEAN PATENT SPECIFICATION

(11) **EP 3 476 947 B1**
(45) Date of publication and mention of the grant of the patent: **12.02.2025**
(21) Application number: 18000835.1
(22) Date of filing: 26.10.2018
(51) Int. Cl.: C12Q 1/6881, C12Q 1/6888

(54) **AN ANALYSIS METHOD USED FOR FOREIGN TISSUE DETECTION AND A PRIMER-PROBE SET USED IN THE ANALYSIS METHOD**
ANALYSEVERFAHREN ZUM NACHWEIS VON FREMDGEWEBE UND EIN PRIMER-SONDENSET ZUM EINSATZ BEI DEM ANALYSEVERFAHREN
PROCÉDÉ D'ANALYSE UTILISÉ POUR LA DÉTECTION DE TISSU ÉTRANGER ET ENSEMBLE D'AMORCES-SONDE UTILISÉ DANS LE PROCÉDÉ D'ANALYSE

(30) Priority: 26.10.2017 TR 201716517
(43) Date of publication of application: 01.05.2019
(73) Proprietor: Tubitak, 06100 Ankara (TR)
(72) Inventor: Yayla, Mediha Esra, Istanbul (TR); Karakaya, Fatih, 41470 Gebze, Kocaeli (TR); Savsar, Serkan, 41470 Gebze, Kocaeli (TR)

(56) References cited:
- EP-A1- 2 077 327
- WO-A2-2012/011085
- JOHN J DOOLEY ET AL: "Detection of meat species using TaqMan real-time PCR assays", MEAT SCIENCE., vol. 68, no. 3, 1 November 2004 (2004-11-01), GB, pages 431 - 438, XP055570216, ISSN: 0309-1740, DOI: 10.1016/j.meatsci.2004.04.010
- B. NOWAK ET AL: "Detection of Bovine Central Nervous System Tissue in Liver Sausages Using a Reverse Transcriptase PCR Technique and a Commercial Enzyme-Linked Immunosorbent Assay", JOURNAL OF FOOD PROTECTION, vol. 68, no. 10, 1 October 2005 (2005-10-01), US, pages 2178 - 2183, XP055570291, ISSN: 0362-028X, DOI: 10.4315/0362-028X-68.10.2178
- SEYBOLDT C ET AL: "REVERSE TRANSCRIPTION-POLYMERASE CHAIN REACTION ASSAY FOR SPECIES-SPECIFIC DETECTION OF BOVINE CENTRAL NERVOUS SYSTEM TISSUE IN MEAT AND MEAT PRODUCTS", JOURNAL OF FOOD PROTEC, INTERNATIONAL ASSOCIATION FOR FOOD PROTECTION, US, vol. 66, no. 4, 1 April 2003 (2003-04-01), pages 644 - 651, XP008048368, ISSN: 0362-028X

## Description

### Technical Field

The present invention is defined in the appended claims. It relates to an analysis method in which method for molecular detection is used based on an expressed sequence tag (EST -Expressed Sequence Tag) having mRNA and / or expression as the mediator molecule in the formation of protein structures providing the specificity of each tissue and organ for the detection of foreign tissue or tissues, such as offal, which may be found in meat and meat products other than carcass meat and relates to a primer-probe set used in an analysis method.

### Prior Art

Nowadays, foreign tissues (fraudulent adulteration tissues, especially from offal tissues) such as offal tissue and so on are added in the production process of meat and meat products because of cost reduction. Although competent authorities in each country make decisions to prevent the involvement of foreign tissues in meat and meat products, it is not deterrent because of the difficulty of detecting foreign tissues involvement and insufficient control. In our country, Turkey, it is prohibited to add bone, cartilage and offal in the production of raw red meat, minced meat and prepared red meat mixtures according to the Turkish Food Codex Meat and Meat Products Announcement. In the same announcement, the addition of bone, cartilage and offal is prohibited in the production of raw poultry meat, turkey minced meat and prepared poultry meat mixtures. It is stated in the announcement that meat products can be prepared from carcass or offal and it is stated that offal meat other than carcass meat can only add to tongue-added salami.

Mixed meat and meat products with offal and foreign tissues can only be determined using histological analysis. However, there is not enough expert staff to be able to apply this analysis efficiently and it is problematic that the method cannot be regarded as numerical output. The expert can only apply this method, and the results are achieved according to their ability to detect the presence of the tissue under the microscope. The result might be subjective. For this, there is a need for systems that can provide objective and precise results that do not require expertise not available in the applications used in the prior art.

Turkish patent document number TR201006092 mentions a kit containing the original primer-probe set used for detection of donkey meat, which can be found in meat products, by Real-Time PCR (RT-PCR) TaqMan probe technique. Invention as mentioned before, the contamination of donkey meat up to 0.1 picogram can be detected in meat products. The kit specific to the donkey type do not make a cross-reaction until the 40 cycles to horse, pig, cattle, sheep, poultry and turkey species; so, the detection of donkey meat is provided in raw and heat-treated meat mixtures.

International patent document number WO2007119066 mentions a kit which allows detection of animal tissues from meat, especially in foods, using PCR technique. Dooley J. et al., Meat Science, 68, pp.431-438, 2004, describes the use f the cytb gene for detecting raw meat admixtures. Nowak B. et al., J. Food Protection, 68, pp.2178-2183, 2005, describes a method for detecting bovine CNS tissue in sausages. Seyboldt C. et al., J. Food Protection, 66, pp.644-651, 2003, describes the use of the GFAP gene for detecting bovine CNS tissue in meat products.WO2012011085 A2 describes donkey specific primers and probes for evaluating beef products. EP2077327 A1 describes tissue and organ specific oligonucleotides of an organism.

In order to reduce the cost of meat, minced meat, emulsified and heat-treated meat products, low-quality raw materials are used, and other tissues and organs other than carcass meat can be added in meat products. Therefore, in order to supply healthy and safe food to the consumers and prevent the unfair competition between producer companies, foreign tissues must be detected in meat and meat products. Therefore, an analytical, fast and objective method must be in need for foreign tissue detection.

### Brief Description of Invention

The purpose of this invention is to provide an analysis method for the detection of foreign tissues, such as offal in meat and meat products, using molecular biology and genetic techniques, based on the messenger ribonucleic acid (mRNA) and / or the expressed sequence tags (EST) of the gene sequences with expression, respectively, reverse-transcriptase polymerase chain (PCR) reaction and RT (Real Time)- PCR analysis.

The other purpose of the present invention is to carry out an analysis method in which undesired tissues in meat products are detected by selecting mRNAs which are not found in carcass tissues but which are commonly found in foreign tissues targeted for detection by using databases of one or more mRNA sequences.

### Detailed Description of Invention

An analysis method used for foreign tissues detection the subject of the invention includes the steps of
- Selection of specific mRNA and /or ESTs (Expressed Sequence Tag) to foreign tissues other than the sample to determine of unknown one,
- RNA isolation from the sample for detection of foreign tissues,
- Conversion of isolated RNA into cDNA by reverse transcriptase PCR reaction,
- Amplification of cDNAs by using RT-PCR method with a primer-probe set specific to the selected mRNA and examination of the signal received during the replication reaction to determine whether there is a target tissue in the case of an amplification curve.

In the method of analysis subject of the invention, in the first instance gene indexes of animal species are at for the detection of offal and foreign tissues in meat and meat products and expressed sequence tags (EST) having expression at the unwanted tissues in meat products to be determined. ESTs used in the method of analysis subject of the invention can be found in the databases as complementary sequences or cDNA of mRNAs. Considering the ESTs of a tissue in the gene index, it is shown which tissues the same region is expressed, and there is no need to select a singular EST for each tissue at this count.

In the method of analysis, subject of the invention; at least one EST is detected which includes tissues other than muscle and fat tissue for foreign tissuesdetection in meat and meat products. Preferably, the selected ESTs are seen at high concentrations in tissues that are preferably except for muscle and fat tissue to provide foreign tissue detection in meat and meat products. In another preferred application, results can be ensured by selecting low concentration ESTs in the analysis method.

In the preferred application of the method of analysis subject of the invention, for the detection of offal and foreign tissues in meat and meat products, it is possible to individually make each animal species, and in the case of different animal species, if the same ESTs are found, the offal and/or unknown textures detection can be made in more than one animal specifies.

In the method of analysis subject of the invention, total RNA or mRNA is isolated on the sample to be subjected to foreign tissues detection on the processed or unprocessed meat product after EST selection. Isolated mRNAs or mRNAs on total RNA are translated into complementary DNA (cDNA) by reverse transcriptase enzyme in reverse-transcriptase PCR reaction. In the preferred application of the invention, the primer-probe set or sets that are designed according to thermodynamic rules using EST databases for ESTs present in tissues other than muscle and fat tissue and are active for RT-PCR analysis, used in the analysis method like any above claims for foreign tissues detection in meat and meat products. The primer-probe set or sets used in the analysis method subject of invention are tagged with the same dye ("-tag") in the preferred application for the detection of foreign tissue presence in the result of analysis. In another preferred application, the primer-probe set or sets are tagged with different dyes for detection of different tissues by signals from different sets or sets.

In the analysis method, RT-PCR analysis is performed using primer-probe set or sets to try to find foreign tissues to be detected in the meat product. In this analysis method, whether foreign tissue in the carcass meat of the meat product, each primer-probe set is linked to the corresponding EST region and signals whether any or one of the foreign tissues is present. The analysis method detects the presence of unknown texture when the primer-probe sets are tagged with the same dye and a signal is obtained as the analysis result. The analysis method examines the different signals coming from different primer-probe sets in the case where the primer-probe sets are tagged with different dyes and the signal is obtained as an analysis result, and it determines which foreign tissues are present.

In a sample application of the analysis method, the *Bos taurus* (cattle) mRNA (EST) database is scanned using the www.nebi.nlm.gov/unigene web site to find out mRNAs in which the offal and foreign tissues except for the muscle and fat tissue are present at high concentration. The subject database shows the synthesizing rates of mRNAs in each tissue. As an example, IGLL1 and CSN2 mRNAs synthesized in tissues possibly to make adulteration especially by adding to the meat products but not synthesized in muscle and fat tissue were selected in the database. The sequences of the selected ESTs are exported from the database to design a primer-probe set suitable for the region that is expected to be unique for comparation to the different animal species and other EST sequences in the cattle species. Preferably the primer designs are made based on www.ncbi.nlm.nih.gov/tools/primer-blast/ site and designs are structured on www.idtdna.com site.

After total RNA isolation from meat and meat products, mRNAs in total RNA are converted to cDNA by reverse transcriptase enzyme using polyA primers. The expression of the selected mRNAs in each case on each tissue and the amount of normalized mRNA are shown in Table 1. For IGLL1 and CSN2, the mRNA primer-probe sets are designed as in Tables 2 and 3. Using the primer-probe sets designed according to the IGLL1 and CSN2 EST sequences, after mRNA selection, three researchers have repeated analysis of the assay three times on different days and all are used in the validation of RT-PCR analyzes. As the result of real-time PCR analysis, values obtained under the 35 cycles (C_{T}) indicate that tissue was detected. (C_{T} value in said table shows the number of repeated cycles in RT-PCR analyzes.)

Muscle and adipose (fat) tissues do not show any amplification curve before 35 C_{T}. It validates that the analysis is correct by three different researchers.

In order to be used in the validation study, foreign tissues were supplied from the veterinary controlled slaughterhouse. Samples containing 1% offal and foreign tissue compared to carcass meat were prepared. Specimens have been proven to detect lung, brain, spleen, tongue, heart, liver, stomach (omasum-intestine), breast, ovarium and uterine tissues. It is possible to detect up to 1% detection limit of other tissues except heart tissue by this analysis method. In addition, when the tissues are subjected to heat treatment at 72°C for about 3 hours with addition of sodium salt, potassium salt, sugar and spices, including mixtures up to the 1% detection limit, there is no change in results, and offal and foreign tissues are detected.

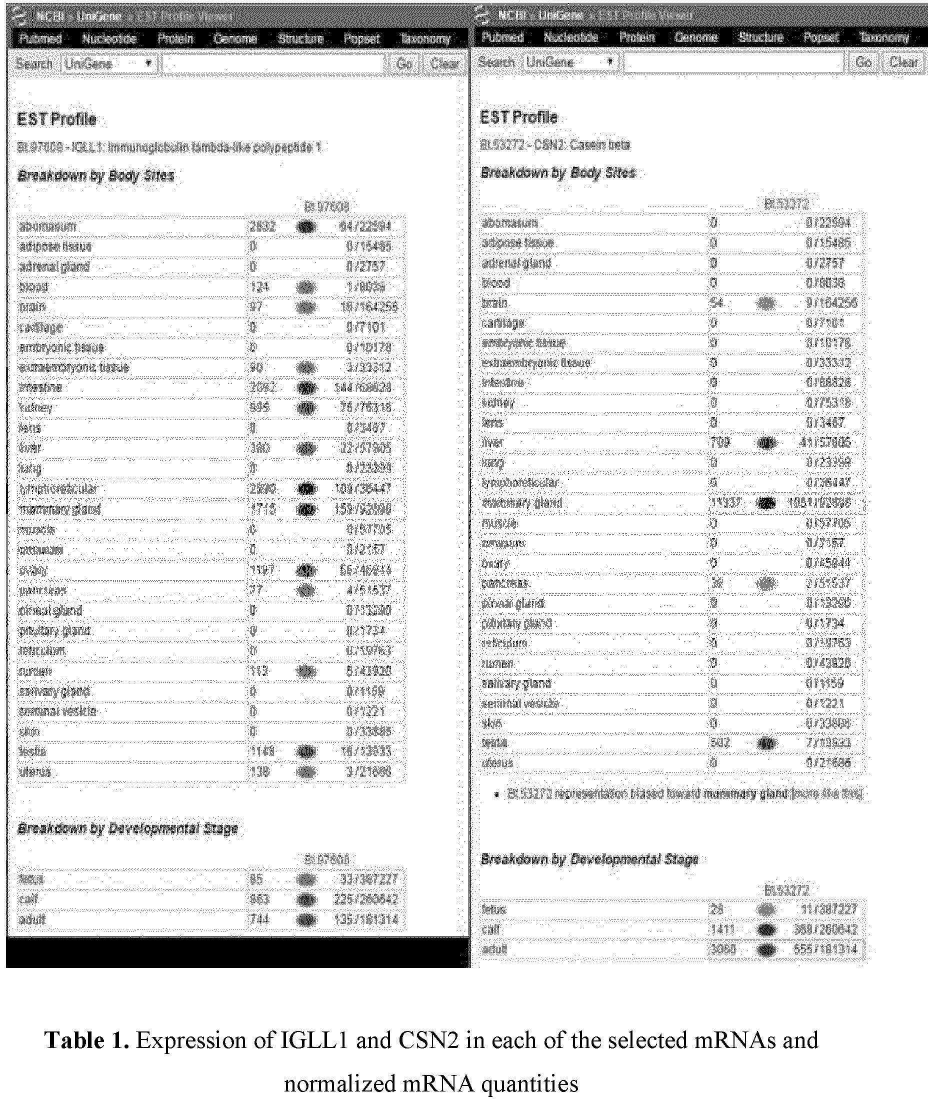

**Table 2. Primer-probe set values performed for IGLL1 mRNA**

| | **Start** | **Stop** | **Length** | **Tm** | **GC%** |
|---|---|---|---|---|---|
| Forward | 250 | 270 | 20 | 64 | 50 |
| **TTCGATTTCCAGTCGCTGCT** | | | | | |
| Probe | 300 | 324 | 24 | 68 | 54 |
| **CTTGCTGTTGCTCTGTTTGGAGGC** | | | | | |
| Reverse | 539 | 559 | 20 | 64 | 55 |
| **TGCAGCTGGTGACTACAGTG** | | | | | |

**Table 3. Primer-probe set values performed for CSN2 mRNA**

| | **Start** | **Stop** | **Length** | **Tm** | **GC%** |
|---|---|---|---|---|---|
| Forward | 437 | 457 | 20 | 65 | 55 |
| **GGGATGTTTTGTGGGAGGCT** | | | | | |
| Probe | 533 | 557 | 24 | 68 | 50 |
| **TCCTCTGTTTGCTGCTGTTCCTCA** | | | | | |
| Reverse | 573 | 595 | 22 | 63 | 40.9 |
| **AGCCTTTCAAGCAGTGAGAAAA** | | | | | |

**Table 4. Cycle (C_{T}) values provided by three researchers from this analysis method on three times, different days.**

| **Sample name** | **User** | **1.run C_{T} value** | **2.run C_{T} value** | **3.run C_{T} value** | **Average C_{T} value** |
|---|---|---|---|---|---|
| %1 STOMACH | User-1 | 29,95 | 30,13 | 30,8 | 30,13 |
| %1 TONGUE | User-1 | 30,8 | 30,92 | 31,25 | 30,92 |
| %1 BREAST | User-1 | 30,61 | 30,1 | 30,71 | 30,61 |
| %1 SPLEEN | User-1 | 32,26 | 32,28 | 32,17 | 32,26 |
| %1 OVARIUM | User-1 | 28,14 | 28,14 | 28,44 | 28,14 |
| %1 UTERUS | User-1 | 33,99 | Undetermined | 33,69 | 33,99 |
| %1 BRAIN | User-1 | Undetermined | 32,53 | 33,18 | 33,18 |
| %1 HEART | User-1 | Undetermined | Undetermined | Undetermined | Undetermined |
| %1 LUNGS | User-1 | 33,1 | 32,81 | 32,62 | 32,81 |
| %1 LIVER | User-1 | 30,8 | 30,67 | 31 | 30,8 |
| %5 MIXTURE | User-1 | 29,68 | 28,77 | 28,9 | 28,9 |
| STOMACH1 (tripe) | User-1 | 33,49 | 31,88 | 33,59 | 33,49 |
| STOMACH2 (gizzard) | User-1 | 33,69 | 33,46 | Undetermined | 33,57 |
| STOMACH3 (psalterium) | User-1 | 31,89 | Undetermined | Undetermined | Undetermined |
| STOMACH4 (omasum) | User-1 | 25,62 | Undetermined | Undetermined | Undetermined |
| STOMACH (omasum-intestine) | User-1 | 31,63 | 31,64 | 31,9 | 31,64 |
| TONGUE | User-1 | 33,28 | 32,43 | 32,91 | 32,91 |
| BREAST | User-1 | 15,13 | 15,45 | 16,11 | 15,45 |
| SPLEEN | User-1 | 33,02 | 32,9 | 33,09 | 33,02 |
| OVARIUM | User-1 | 32,49 | 32,34 | 32,36 | 32,36 |
| UTERUS | User-1 | 25,59 | 25,27 | 25,47 | 25,47 |
| BRAIN | User-1 | 32,2 | 31,79 | 32,23 | 32,2 |
| HEART | User-1 | 30,4 | 28,98 | 30,06 | 30,06 |
| LUNGS | User-1 | 29,95 | 30,29 | 29,71 | 29,95 |
| TESTIS | User-1 | 34,07 | 33,26 | 34,07 | 34,07 |
| KIDNEY | User-1 | 33,5 | 34,01 | 34,05 | 34,01 |
| LIVER | User-1 | 32,43 | 31,56 | 31,2 | 31,56 |
| STRIPED MUSCLE | User-1 | Undetermined | Undetermined | Undetermined | Undetermined |
| FAT | User-1 | Undetermined | Undetermined | Undetermined | Undetermined |
| NO TEMPLATE | User-1 | Undetermined | Undetermined | Undetermined | Undetermined |
| %1 STOMACH | User-2 | 30,7 | 29,89 | 30,28 | 30,28 |
| %1 TONGUE | User-2 | 31,71 | 29,83 | 30,58 | 30,58 |
| %1 BREAST | User-2 | 30,25 | 29,55 | 30,06 | 30,06 |
| %1 SPLEEN | User-2 | 33,73 | 30,42 | 31,71 | 31,71 |
| %1 OVARIUM | User-2 | 27,62 | 27,25 | 26,92 | 27,25 |
| %1 UTERUS | User-2 | 33,83 | 31,3 | 32,51 | 32,51 |
| %1 BRAIN | User-2 | 32,06 | 31,12 | 31,64 | 31,64 |
| %1 HEART | User-2 | Undetermined | Undetermined | Undetermined | Undetermined |
| %1 LUNGS | User-2 | 31,98 | 31,93 | 32,14 | 31,98 |
| %1 LIVER | User-2 | 30,86 | 30,37 | 30,66 | 30,66 |
| %5 MIXTURE | User-2 | 29,15 | 28,56 | 28,61 | 28,61 |
| STOMACH1 (tripe) | User-2 | Undetermined | 31,65 | 32,94 | 32,30 |
| STOMACH2 (gizzard) | User-2 | Undetermined | 33,49 | Undetermined | Undetermined |
| STOMACH3 (psalterium) | User-2 | Undetermined | Undetermined | 33,73 | Undetermined |
| STOMACH4 (omasum) | User-2 | 26,13 | 33,6 | 32,17 | 32,17 |
| STOMACH (omasum-intestine) | User-2 | 30,95 | 29,4 | 30,03 | 30,03 |
| TONGUE | User-2 | 31,48 | 30,03 | 29,79 | 30,03 |
| BREAST | User-2 | 15,24 | 14,72 | 14,82 | 14,82 |
| SPLEEN | User-2 | 32,94 | 31,75 | 31,32 | 31,75 |
| OVARIUM | User-2 | 33 | 31,3 | 31,12 | 31,3 |
| UTERUS | User-2 | 25,55 | 24,82 | 25,16 | 25,16 |
| BRAIN | User-2 | 33,96 | 32,63 | 31,24 | 32,63 |
| HEART | User-2 | 31,43 | 30,92 | 31,28 | 31,28 |
| LUNGS | User-2 | 30,7 | 29,25 | 29,23 | 29,25 |
| TESTIS | User-2 | Undetermined | Undetermined | Undetermined | Undetermined |
| KIDNEY | User-2 | Undetermined | 32,04 | 31,91 | 31,98 |
| LIVER | User-2 | 30,96 | 29,96 | 30,16 | 30,16 |
| STRIPED MUSCLE | User-2 | Undetermined | Undetermined | Undetermined | Undetermined |
| FAT | User-2 | Undetermined | Undetermined | Undetermined | Undetermined |
| NO TEMPLATE | User-2 | Undetermined | Undetermined | Undetermined | Undetermined |
| %1 STOMACH | User-3 | 30,39 | 30,41 | 30,82 | 30,41 |
| %1 TONGUE | User-3 | 31,46 | 31,27 | 31,3 | 31,3 |
| %1 BREAST | User-3 | 30,4 | 30,58 | 30,37 | 30,4 |
| %1 SPLEEN | User-3 | 31,83 | 32,04 | 31,45 | 31,83 |
| %1 OVARIUM | User-3 | 27,44 | 27,59 | 27,99 | 27,59 |
| %1 UTERUS | User-3 | 34,08 | 33,74 | 32,65 | 33,74 |
| %1 BRAIN | User-3 | 33,34 | 32,16 | 31,9 | 32,16 |
| %1 HEART | User-3 | Undetermined | 32,3 | Undetermined | Undetermined |
| %1 LUNGS | User-3 | 31,35 | 32,08 | 31,67 | 31,67 |
| %1 LIVER | User-3 | 31,1 | 29,54 | 30,46 | 30,46 |
| %5 MIXTURE | User-3 | 29,01 | 28,75 | 28,89 | 28,89 |
| STOMACH1 (tripe) | User-3 | 32,93 | Undetermined | 33,57 | 33,25 |
| STOMACH2 (gizzard) | User-3 | Undetermined | Undetermined | Undetermined | Undetermined |
| STOMACH3 (psalterium) | User-3 | Undetermined | Undetermined | 33,73 | Undetermined |
| STOMACH4 (omasum) | User-3 | Undetermined | Undetermined | 32,69 | Undetermined |
| STOMACH (omasum-intestine) | User-3 | 30,95 | 30,65 | 30,28 | 30,65 |
| TONGUE | User-3 | Undetermined | 31,94 | 31,13 | 31,54 |
| BREAST | User-3 | 16,95 | 15,95 | 15,41 | 15,95 |
| SPLEEN | User-3 | 32,43 | 32,19 | 31,37 | 32,19 |
| OVARIUM | User-3 | 30,78 | 30,48 | 30,63 | 30,63 |
| UTERUS | User-3 | 25 | 24,55 | 24,83 | 24,83 |
| BRAIN | User-3 | 32,7 | 32,72 | 31,37 | 32,7 |
| HEART | User-3 | Undetermined | 31,14 | 31,11 | 31,13 |
| LUNGS | User-3 | 30,16 | 30,07 | 29,98 | 30,07 |
| TESTIS | User-3 | Undetermined | 33,96 | Undetermined | Undetermined |
| KIDNEY | User-3 | Undetermined | 33,53 | 32,81 | 33,17 |
| LIVER | User-3 | Undetermined | 33,59 | 32,76 | 33,18 |
| STRIPED MUSCLE | User-3 | Undetermined | Undetermined | Undetermined | Undetermined |
| FAT | User-3 | Undetermined | Undetermined | Undetermined | Undetermined |
| NO TEMPLATE | User-3 | Undetermined | Undetermined | Undetermined | Undetermined |

All about these basic concepts, it is possible to develop a wide variety of applications related to an analysis method detected for foreign tissue subject of the invention and a primer-probe set used in the method of analysis, and the invention cannot be limited to the examples described herein, it is exactly specified in claims.

## Claims

1. A primer probe sets for offal tissue detection in meat and meat products comprises the *Bos taurus* IGLL1 and CSN2 mRNAs specific nucleotide oligo-primers as:
IGLL1- Forward
TTCGATTTCCAGTCGCTGCT
IGLLI-Probe
CTTGCTGTTGCTCTGTTTGGAGGC
IGLL1-Reverse
TGCAGCTGGTGACTACAGTG
CSN2-Forward
GGGATGTTTTGTGGGAGGCT
**CSN2-Probe**
TCCTCTGTTTGCTGCTGTTCCTCA
**CSN2-Reverse**
AGCCTTTCAAGCAGTGAGAAAA

2. An analysis method for detecting *Bos taurus* offal tissues in meat and meat products by the primer probe sets according to claim 1 comprising the steps of;
- Isolating mRNA from a meat sample
- Converting the isolated mRNA into cDNA by reverse transcriptase PCR reaction,
- Amplifying cDNAs by using Real Time-PCR method with the designed primer-probe sets according to claim 1
- Examining a signal received during the replication reaction

3. The analysis method according to Claim 2 **characterized in** with detecting the presence of offal, tissue when the primer-probe sets are tagged with the same dye and a signal is obtained as the analysis result.

4. The analysis method according to Claim 2 **characterized in** with examining the different signals coming from different primer-probe sets in the case where the primer-probe sets are tagged with different dyes and the signal is obtained as an analysis result, and it determines which offal are present.

## Patentansprüche

1. Ein Primer-Sondensatz für den Nachweis von Abfallgewebe in Fleisch und Fleischprodukten umfasst die *Bos taurus* IGLL1 und CSN2 mRNA-spezifischen Nukleotid-Oligoprimer:
**IGLL1- Vorwärts**
TTCGATTTCCAGTCGCTGCT
**IGLL1-Sonde**
CTTGCTGTTGCTCTGTTTGGAGGC
**IGLL1-Rückwärts**
TGCAGCTGGTGACTACAGTG
**CSN2-Vorwärts**
GGGATGTTTTGTGGGAGGCT
**CSN2-Sonde**
TCCTCTGTTTGCTGCTGTTCCTCA
**CSN2-Rückwärts**
AGCCTTTCAAGCAGTGAGAAAA

2. Analyseverfahren zum Nachweis von *Bos taurus* Abfallgeweben in Fleisch und Fleischprodukten mit den Primer-Sondensätzen nach Anspruch 1, das die folgenden Schritte umfasst:
- Isolierung von mRNA aus einer Fleischprobe
- Umwandlung der isolierten mRNA in cDNA durch reverse Transkriptase-PCR-Reaktion,
- Amplifizieren von cDNAs unter Verwendung des Real Time-PCR-Verfahrens mit den entworfenen Primer-Sondensätzen gemäß Anspruch 1
- Untersuchen eines während der Replikationsreaktion empfangenen Signals

3. Analyseverfahren nach Anspruch 2, **dadurch gekennzeichnet, dass** das Vorhandensein von Abfallgewebe nachgewiesen wird, wenn die Primer-Sondensätze mit demselben Farbstoff markiert sind und ein Signal als Analyseergebnis erhalten wird.

4. Analyseverfahren nach Anspruch 2, **dadurch gekennzeichnet, dass** die verschiedenen Signale, die von verschiedenen Primer-Sondensätzen stammen, in dem Fall untersucht werden, in dem die Primer-Sondensätze mit verschiedenen Farbstoffen markiert sind und das Signal als ein Analyseergebnis erhalten wird, und dass bestimmt wird, welche Abfälle vorhanden sind.

## Revendications

1. Un ensemble de sondes d'amorces pour la détection de tissus d'abats dans la viande et les produits à base de viande comprend les oligoamorces nucléotidiques spécifiques des ARNm IGLL1 et CSN2 de *Bos taurus,* comme suit :
**IGLL1- Avant**
TTCGATTTCCAGTCGCTGCT
**IGLL1-Sond**e
CTTGCTGTTGCTCTGTTTGGAGGC
**IGLL1-Inverse**
TGCAGCTGGTGACTACAGTG
**CSN2-Avant**
GGGATGTTTTGTGGGAGGCT
**CSN2-Sonde**
TCCTCTGTTTGCTGCTGTTCCTCA
**CSN2-Inverse**
AGCCTTTCAAGCAGTGAGAAAA

2. La méthode d'analyse pour détecter les tissus d'abats de *Bas taurus* dans la viande et les produits à base de viande à l'aide des ensembles de sondes d'amorces selon la revendication 1, comprenant les étapes suivantes ;
- Isolation de l'ARNm à partir d'un échantillon de viande
- Conversion de l'ARNm isolé en ADNc par transcription inverse de la réaction PCR,
- Amplification des ADNc par la méthode de PCR en temps réel avec les ensembles amorce-sonde conçus selon la revendication 1
- Examen d'un signal reçu lors de la réaction de réplication

3. La méthode d'analyse selon la revendication 2 est **caractérisée par** la détection de la présence de tissus d'abats lorsque les ensembles amorce-sonde sont marqués avec le même colorant et qu'un signal est obtenu comme résultat de l'analyse.

4. La méthode d'analyse selon la revendication 2 est **caractérisée en ce qu'**elle examine les différents signaux provenant de différents ensembles d'amorces-sondes dans le cas où les ensembles d'amorces-sondes sont marqués avec différents colorants et le signal est obtenu comme résultat d'analyse, et elle détermine les abats qui sont présents.
